# DEMANDE DE BREVET EUROPEEN

(11) **EP 3 366 351 A1**
(43) Date de publication de la demande: **29.08.2018**
(21) Numéro de dépôt: 18157963.2
(22) Date de dépôt: 21.02.2018
(51) Int. Cl.: A61Q 3/02, A61K 8/73, A61K 8/85

(54) **MÉTHODE DE STABILISATION DE LA COLORATION DE VERNIS À ONGLES**

(30) Priorité: 23.02.2017 LU 100108
(71) Demandeur: International Lacquers S.A., 3225 Bettembourg (LU)
(72) Inventeur: DYLEWICZ, Carole, 57180 Terville (FR); EDDOUMY, Fatima, 57840 Ottange (FR); DEROSIER, Frédéric, 57680 Corny sur Moselle (FR)
(74) Mandataire: Office Freylinger

(57) **Abrégé**

La présente invention concerne l'utilisation d'une résine de copolymère(s) d'acide adipique, d'acide fumarique, de néopentylglycol et de cyclohexanediméthanol, de préférence une résine de Polyester-26 comme agent(s) de protection contre les UV dans une composition anhydre de vernis à ongles.

## Description

### Domaine technique

La présente invention concerne les vernis à ongles et plus particulièrement les ingrédients pour vernis à ongles offrant un vernis à ongles stable aux rayons UV.

### Etat de la technique

Les vernis à ongles sont généralement utilisés pour maquiller les ongles ainsi que les faux ongles. Les vernis à ongles sont habituellement composés d'un agent filmogène, d'un solvant, et éventuellement d'un ou plusieurs plastifiants, d'une ou plusieurs résines, d'un ou plusieurs agents de rhéologie et un ou plusieurs colorants.

Pour répondre aux exigences des utilisateurs, les vernis à ongles doivent satisfaire plusieurs critères :
- une bonne application, lorsque le vernis à ongles est appliqué sur l'ongle, il doit former une surface lisse, sans stries avec une épaisseur constante afin d'obtenir un film et une coloration homogènes,
- un étalement facilité sur toute la surface de l'ongle pour rendre son utilisation pratique,
- une brillance accrue et qui persiste dans le temps,
- un temps de séchage et de durcissement sur l'ongle très courts,
- une bonne tenue du vernis à ongles sur l'ongle, il est en effet souhaité que le vernis s'écaille le moins possible afin de conserver un aspect esthétique,
- un flacon de vernis à ongles stable aux rayons UV lorsque exposé en vitrine, et/ou salle de bain, etc.

Les vernis à ongle sont soumis aux conditions extérieures. La chaleur et la lumière sont deux facteurs qui accélèrent la décomposition des composants du vernis à ongle. Sur l'ongle ou lorsque le flacon transparent est exposé, la température et la lumière accélèrent la décoloration des pigments et accélère le vieillissement des ingrédients comme l'agent filmogène (nitrocellulose) et/ou les résines.

De nouvelles formulations sont sans cesse développées pour remédier à ces inconvénients. D'une manière générale on observe cependant que les nouvelles formulations en deviennent de plus en plus complexes, car tout remède à un inconvénient risque de produire d'autres difficultés. En effet, la modification d'une composition existante en vue d'en améliorer une propriété se fait en général soit par ajout d'un composant supplémentaire donnant cette propriété, soit, si l'effet négatif sur ladite propriété provient d'un composant de la formulation connue, par la substitution de ce composant par un autre composant n'ayant pas ou moins cet effet négatif. Cependant, indépendamment de la solution choisie il est probable que l'ajout ou la substitution d'un composant affecte négativement d'autres propriétés de la formulation.

Par exemple, dans le but de protéger la formulation ou les agents colorants de la lumière UV, des absorbants UV sont classiquement utilisés dans les produits cosmétiques. Ces absorbants UV sont soit d'origine chimique (organique) ou minérale (inorganique). Parmi les absorbants UV utilisables dans les compositions de vernis à ongles, on peut citer par exemple le dioxyde de titane ou l'oxyde de zinc ainsi que les benzophénones. L'inconvénient majeur du dioxyde de titane est l'effet de blanchiment qu'il induit lors de son adjonction à une matrice organique. Les benzophénones, quant à elles, sont parfois contestées, parce qu'elles pourraient entrainer des allergies et sont instables d'où le problème de leur mise en formulation.

La demande internationale WO 2015/002620 A1 divulgue une composition d'un vernis à ongles, qui brille de lui-même dans un environnement sans éclairage (nuit), qui possède une toxicité diminuée et une résistance aux UV accrue. Le vernis à ongles qui y est décrit est constitué d'un nombre élevé de d'ingrédients, parmi lesquels l'huile de jojoba est présente notamment pour diminuer les effets négatifs de la lumière UV et ainsi augmenter la durée de vie de la composition.

En conséquence, il existe notamment le besoin de développer des vernis à ongles qui ont une résistance accrue à la chaleur et la lumière UV, mais de préférence sans en altérer (significativement) leurs autres propriétés importantes, notamment la tenue, la brillance, la couvrance, le temps de séchage ou le durcissement.

### Objet de l'invention

Un objet de la présente invention est par conséquent de proposer une protection UV pour les vernis à ongles qui ne compromet pas ou peu les autres propriétés de la formulation.

### Description générale de l'invention

Afin de résoudre le problème mentionné ci-dessus, la présente invention propose une utilisation d'une résine de copolymère(s) d'acide adipique, d'acide fumarique, de néopentylglycol et de cyclohexanediméthanol comme agent(s) de protection contre les UV (encore appelés absorbants UV) dans une composition anhydre de vernis à ongles. De préférence, le ou les copolymères sont des copolymères statistiques. Parmi ces résines de copolymères, l'utilisation de Polyester-26 (Polyester-26, désignation INCI) est particulièrement préférée. Dans une variante préférée, la quantité de résine de copolymères d'acide adipique, d'acide fumarique, de néopentylglycol et de cyclohexanediméthanol utilisée pour obtenir cet effet est comprise entre 0,1 à 5% et de préférence entre 1 à 3% en poids sec par rapport au poids total de la composition anhydre.

Dans une variante particulièrement préférée, la résine de copolymères d'acide adipique, d'acide fumarique, de néopentylglycol et de cyclohexanediméthanol est solubilisée dans un solvant. De préférence, le solvant utile pour solubiliser la résine à base d'un ou de plusieurs copolymères d'acide adipique, d'acide fumarique, de néopentylglycol et de cyclohexanediméthanol et en particulier la résine de Polyester-26, est l'acétate d'éthyle ou l'acétate de butyle, de préférence l'acétate de butyle. Préférablement, la résine est solubilisée dans le solvant dans les proportions suivantes :
- 60 % en poids d'une résine à base d'un ou de plusieurs copolymères d'acide adipique, d'acide fumarique, de néopentylglycol et de cyclohexanediméthanol, de préférence d'une résine de Polyester-26 et
- 40 % en poids de solvant, de préférence d'acétate de butyle, par rapport au poids total de la résine solubilisée dans le solvant.

De façon surprenante, il a été mis en évidence que la résine de copolymères d'acide adipique, d'acide fumarique, de néopentylglycol et de cyclohexanediméthanol, et en particulier la résine de Polyester-26, permettait une protection contre les UV en plus d'améliorer considérablement les propriétés d'étalement et d'application du vernis à ongles. L'utilisation de ces résines dans une composition anhydre pour vernis à ongles conduit alors à une pose rapide et pratique du vernis à ongles et permet une décoloration beaucoup moins prononcée au cours du temps, en fonction de la température et de l'ensoleillement. De surcroît, l'ajout de ces résines n'affecte pas significativement les autres propriétés de la composition anhydre pour vernis à ongles. Ainsi, l'utilisation de ces résines de copolymères d'acide adipique, d'acide fumarique, de néopentylglycol et de cyclohexanediméthanol, en particulier la résine de Polyester-26, conduit à l'obtention d'une composition anhydre pour vernis à ongle qui offre une bonne protection aux UV et qui est de surcroît facile à appliquer sur l'ongle et présente une tenue, une brillance, une couvrance ainsi qu'un temps de séchage et de durcissement avantageux.

En outre, l'utilisation de résine de copolymères d'acide adipique, d'acide fumarique, de néopentylglycol et de cyclohexanediméthanol, en particulier la résine de Polyester-26, présente l'avantage qu'il offre une très bonne compatibilité avec les autres composants habituellement utilisés dans les compositions anhydres. Il peut donc être utilisé dans tous les types de compositions anhydres pour vernis à ongles comme par exemple les vernis à ongles à effet (miroir, craquelé, irisé, martelé, magnétique, mat, métallisé, etc.), les vernis à ongles longue tenue « long lasting », vernis dits « gel like », mais également les vernis à ongles dits « top coat » ou « base coat » (destinés à être appliqués sur ou en dessous d'un vernis à ongles). Dans tous les cas, l'utilisation de résine de copolymères d'acide adipique, d'acide fumarique, de néopentylglycol et de cyclohexanediméthanol, en particulier de la résine de Polyester-26, résultera en plus d'une amélioration significative des propriétés d'étalement et d'application du vernis sur l'ongle, d'une protection contre les rayonnements UV sans pour autant affecter les autres propriétés (esthétiques ou autres) du vernis à ongles. Par exemple, dans le cas d'une formulation « long lasting », la tenue du vernis à ongles ne sera pas affectée par l'ajout de la résine de copolymères d'acide adipique, d'acide fumarique, de néopentylglycol et de cyclohexanediméthanol, en particulier de la résine de Polyester-26.

De façon préférée, la résine est utilisée au sein d'une composition anhydre qui comprend au moins un solvant organique cosmétiquement acceptable et au moins un agent filmogène.

Le ou les agent(s) filmogène(s) compris dans la composition anhydre pour vernis à ongles peu(ven)t être choisi(s) parmi le groupe constitué par la cellulose, un dérivé de cellulose, une résine vinylique, une résine acrylique et n'importe quelle combinaison de ceux-ci. Les dérivés de cellulose sont par exemple la nitrocellulose, l'acétate butyrate cellulose, l'éthylcellulose et l'hydroxypropylcellulose. Préférentiellement, on choisira la nitrocellulose comme agent filmogène.

Un agent filmogène seul ou une combinaison d'agents filmogènes peut être utilisé(e) dans la composition anhydre pour vernis à ongles dans les proportions allant de 10 % à 20 % en poids sec et de façon préférée de 12 % à 16 % en poids sec par rapport au poids total de la composition.

Selon un mode de réalisation de l'invention, la composition anhydre pour vernis à ongles comprend un solvant organique, en plus du solvant éventuellement utilisé pour solubiliser la résine de copolymères d'acide adipique, d'acide fumarique, de néopentylglycol et de cyclohexanediméthanol, en particulier la résine de Polyester-26. Ce solvant organique peut être choisi parmi le groupe constitué par le toluène, le xylène, l'acétate de méthyle, l'acétate d'éthyle, l'acétate de butyle, l'acétate d'isobutyle, l'acétate de propyle, une cétone, un ester, un alcool, tel que l'éthanol dénaturé (avec la méthyléthylcétone ou le diéthyléther), l'isopropanol, le butanol, le diacétone alcool, les hydrocarbures linéaires ou cycliques, comme par exemple l'hexane, le cyclohexane, l'heptane et n'importe quelle combinaison de ceux-ci.

Le solvant organique pourra être choisi en fonction de ses propriétés physico-chimiques et en considérations des autres composants présents dans la composition anhydre pour vernis à ongles. En outre de façon préférée, la quantité de solvant organique présente dans la composition anhydre pour vernis à ongles est comprise entre 60% et 80% en poids par rapport au poids total de la composition.

Avantageusement, le solvant organique est l'acétate d'éthyle et/ou l'acétate de butyle et/ou de l'alcool.

En plus, de la résine de copolymères d'acide adipique, d'acide fumarique, de néopentylglycol et de cyclohexanediméthanol, en particulier la résine de Polyester-26, des résines supplémentaires (différentes) peuvent également être ajoutées dans la composition. En particulier, ces résines supplémentaires permettent d'augmenter le collant et l'adhérence du film sur l'ongle.

Les résines pouvant être utilisées sont les suivantes :
- les résines tosylamide/formaldéhyde ou résines toluènesulfonamide/ formaldéhyde,
- les résines tosylamide/époxy ou résines toluènesulfonamide/époxy,
- copolymères acide adipique/néopentyl glycol/anhydride trimellitique,
- copolymères d'anhydride phtalique/glycérine/glycidyl décanoate,
- copolymères d'anhydride phtalique/anhydride trimellitique/glycols,
- copolymères de glycérine/acide phtalique,
- copolymères styrène/acrylate/acrylonitrile,
- polymères et copolymères d'acrylates,
- copolymères d'acrylates et de styrène,
- sucrose acétate isobutyrate,
- résine polyvinylbutyral.

En plus de leur action collante, la plupart de ces résines additionnelles ont également une action plastifiante. Les résines additionnelles peuvent être utilisées dans la formule, seules ou sous la forme de mélanges dans des quantités allant de 3 à 20 % en poids sec, de préférence de 5 à 15% en poids sec, en particulier de 7 à 12 % en poids sec par rapport au poids total de la composition.

Selon un mode de réalisation, la composition anhydre pour vernis à ongles peut également comprendre des plastifiants. Les plastifiants sont des composés ayant une température d'ébullition élevée qui ne s'évaporent pas lors du séchage du vernis à ongles. Ils sont utilisés pour moduler la dureté du film formé afin d'obtenir les caractéristiques physico-chimiques du film souhaitées et représentent en général de 1 à 12 % en poids sec, de préférence 5 à 10 % en poids sec, en particulier de 6 à 8 % en poids sec par rapport au poids total de la composition.

Des exemples de plastifiants utilisables dans l'invention sont l'acétyl tributyl citrate, l'acétyl triéthyl citrate, le tributyl citrate, le triéthylcitrate, le dibutyl phtalate, le triphénylphosphate, la triacétine, le triméthyl pentanyl diisobutyrate, la triéthylhexanoïne, le sucrose benzoate, le dibutyl adipate, le diéthyl phthalate, le diisobutyl adipate, le diisopropyl adipate, le dipropylène glycol dibenzoate, etc.

Pour colorer ou fournir des effets particuliers à la composition anhydre pour vernis à ongles, celle-ci peut comprendre un ou plusieurs agents de coloration choisis parmi les pigments, les nacres, les glitters (paillettes) et/ou les particules métalliques. Ces agents de coloration fournissent l'aspect esthétique recherché par les utilisateurs de vernis à ongles. Ces agents de coloration peuvent produire des effets très différents tels que « irisé », « métallisé », « nacré », « miroir », « crème », « pailleté », craquelé », « mat », « néon », « fluorescent », « holographique », etc.

En particulier, les pigments utilisés peuvent être sélectionnés parmi le groupe constitué par le dioxyde de titane (CI 77891), l'oxyde de fer noir (CI 77499), l'oxyde de fer rouge (CI 77491), le DC Red 6 Ba Lake (CI 15850), le DC Red 7 Ca Lake (CI 15850:1), le DC Red 34 Ca Lake (CI 15880), le FDC Yellow 5 Al Lake (CI 19140), le FDC Blue 1 Al Lake (CI 42090), le bleu ultramarine (CI 77007), le DC Violet 2 (CI 60725), le DC Black 2 (CI 77266), etc.

Les nacres sont également utilisables dans l'invention, les nacres sont des particules minérales de différentes natures recouvertes d'une ou plusieurs couches d'oxydes (oxydes de titane ou oxydes de fer) ou encore de pigments. Les nacres peuvent se présenter sous la forme d'une poudre ou de plaque de tailles variées. Parmi les nacres utilisables dans l'invention, on peut citer :
- les nacres préparées à partir de mica naturel et ayant subi des opérations de coating successives,
- les nacres préparées à partir de mica synthétique appelé également « synthetic fluorphlogopite »,
- les nacres préparées à partir de calcium sodium borosilicate,
- les nacres préparées à partir d'oxyde de silice.

Les glitters (paillettes) utilisables dans l'invention peuvent être préparés à partir de plusieurs types de matériaux comme les films polyesters comportant en surface une couche métallique, le polyéthylène téréphtalate, le polybutylène téréphtalate, les copolymères d'acrylates, les copolymères d'acrylates ou encore l'aluminium.

Selon un mode de réalisation de l'invention, la composition anhydre pour vernis à ongles comprend une quantité d'agent de coloration comprise de 0,1 % à 20 % en poids sec et de préférence de 0,5 % à 12 % en poids sec par rapport au poids total de la composition.

La composition anhydre pour vernis à ongles peut en outre comprendre divers additifs choisis parmi les agents filmogènes, les résines, les agents rhéologiques, les modificateurs de surface et les agents dits « traitants ».

Les différents additifs utilisables dans l'invention sont :
Parmi les additifs de rhéologie, on citera notamment les dérivés d'argile modifiés comme le stéaralkonium hectorite ou le stéaralkonium bentonite ou de type silice pyrogénée. Ces additifs de rhéologie seront de préférence utilisés de 0,1 à 4 % en poids sec par rapport au poids total de la composition. Ces additifs permettent notamment la suspension des particules insolubles dans le vernis tels que les pigments ou encore les nacres.

Si nécessaire ou souhaité, la composition anhydre peut également comporter d'autres absorbeurs UV, de préférence en quantité nettement plus réduite qu'en l'absence de résine de copolymère(s) d'acide adipique, d'acide fumarique, de néopentylglycol et de cyclohexanediméthanol. Parmi ces autres absorbants UV, on citera : la benzophénone-1 (CAS 131-56-6), la benzophénone-3 (CAS 131-57-7), le benzyl salicylate (CAS 118-58-1), l'étocrylène (CAS 5232-99-5), le drométrizole (CAS 2440-22-4), le butyl méthoxydibenzoylméthane (CAS 70356-09-1), etc.

Les autres absorbants UV seront utilisés seuls ou sous la forme de combinaison dont le pourcentage total dans la formule peut aller de 0 à 1,5 % en poids sec par rapport au poids total de la composition.

Parmi les additifs utilisables selon l'invention, on citera également les modificateurs de surface comme les cyclométhicones ou cyclopentasiloxane, les diméthicones et le triméthylsiloxysilicate.

Ces additifs de surface sont généralement utilisés dans des faibles quantités dans la formulation allant de 0,1 à 5 % en poids sec par rapport au poids total de la composition.

On citera également les additifs dits « traitants » de l'ongle utilisables dans la composition selon l'invention. Les additifs traitants peuvent représenter de 0,01 % à 5 % en poids sec, de préférence de 0,05 % à 2 % en poids sec par rapport au poids total de la composition. Parmi ces additifs « traitants », on citera :
- le formaldéhyde utilisé comme durcisseur de l'ongle,
- la vitamine E acétate (tocophéryl acétate),
- la vitamine A palmitate (rétinyl palmitate),
- les dérivés organiques ou inorganiques de calcium tels que le chlorure de calcium, le pantothénate de calcium,
- le diméthyloxobenzodioxasilane,
- la myrrhe,
- les acides aminés soufrés comme la cystéine, ses sels et leurs dérivés, la cystine, le glutathion. Les acides aminés soufrés sont en effet capables de créer des ponts de réticulation entre la kératine et les groupes SH libres de ces dérivés soufrés,
- la kératine hydrolysée d'origine végétale,
- les alpha-hydroxyacides comme l'acide citrique, l'acide ascorbique,
- la biotine,
- l'urée et la diméthylurée.

Tous les modes de réalisation cités précédemment peuvent être combinés sous réserve de leur faisabilité technique.

### Exemples

Les exemples, ci-dessous, concernent diverses formulations de compositions anhydres pour vernis à ongles.

Afin d'évaluer la résistance du polyester-26 à la température et aux UV, six formulations distinctes de teinte rose-pâle et violette (Tableau 1) ont été exposées à la lumière naturelle du jour et à des radiations en laboratoire par un simulateur solaire à arc xénon (modèle: Suntest Atlas). Ces six formulations ont ensuite été appliquées sur une carte de contraste par un bar coater puis analysées au spectromètre. Les mesures au spectromètre permettent de déterminer les paramètres delta Ecmc de chaque formulation et ont été effectuées après deux cycles de 30 h au Suntest (Tableau 2). Le paramètre delta Ecmc est défini comme une mesure de différence entre deux couleurs et a permis de juger le degré de décoloration des formulations après exposition.

A partir d'un delta Ecmc > 0,3, la formulation a été jugée très dégradée par le rayonnement lumineux, entre 0,15 et 0,3, la formulation a été jugée moyennement dégradée et lorsque le delta Ecmc est < 0,15, la formulation a été jugée très résistante aux rayonnements lumineux.

La composition correspondant à la base dite standard ne comprend pas de résine de Polyester-26. Les compositions correspondant aux exemples 1-5 comprennent diverses concentrations de résine de Polyester-26 solubilisée dans de l'acétate de butyle.

**Tableau 1**

| **Ingrédients** | **fonction** | **Base standard** | **Ex 1** | **Ex 2** | **Ex 3** | **Ex 4** | **Ex 5** |
|---|---|---|---|---|---|---|---|
| Acétate d'éthyle | Solvant organique | 34,74 | 34,64 | 34,53 | 34,32 | 34,11 | 33,91 |
| Acétate de butyle | Solvant organique | 27 | 26,92 | 26,84 | 26,68 | 26,51 | 26,35 |
| Alcool isopropylique (provenant de la nitrocellulose) | Solvant | 6,41 | 6,39 | 6,37 | 6,33 | 6,29 | 6,26 |
| Acétyl Tri-butyl Citrate | Plastifiant | 6,73 | 6,71 | 6,69 | 6,65 | 6,61 | 6,57 |
| Nitrocellulose (en poids sec) | Filmogène | 12,62 | 12,58 | 12,54 | 12,47 | 12,39 | 12,32 |
| Phthalic Anhydride / Trimellitic Anhydride / Glycols copolymer (en poids sec) | Résine | 10,5 | 10,47 | 10,44 | 10,37 | 10,31 | 10,25 |
| Bentonite | Agent thixotropant | 2 | 1,99 | 1,99 | 1,98 | 1,96 | 1,95 |
| Polyester-26 (en poids sec) | Agent d'application et d'étalement et de protection contre les UV | 0 | 0,3 | 0,6 | 1,2 | 1,8 | 2,4 |
| Total (% en poids) | | 100 | 100 | 100 | 100 | 100 | 100 |

**Tableau 2**

| **Teinte** | **Base standard** | **Ex 1** | **Ex 2** | **Ex 3** | **Ex 4** | **Ex 5** |
|---|---|---|---|---|---|---|
| Rose pâle | 1,07 | 0,78 | 0,45 | 0,11 | 0,04 | 0.03 |
| Violette | 0,54 | 0,48 | 0,42 | 0,41 | 0,38 | 0.38 |

Il a été observé une décoloration très prononcée de la composition standard rose-pâle alors qu'une très faible voir aucune décoloration apparente n'a été observée pour la composition correspondant aux exemples 3-4-5. L'apparition de légères auréoles bleutées a été observée pour la composition standard violette en comparaison avec la composition correspondant aux exemples 1-5 qui est restée inchangée. Une faible décoloration a néanmoins été observée pour l'ensemble des compositions violettes, celle-ci étant tout de même moins prononcée pour la composition correspondant aux exemples 1-5 en comparaison avec la composition standard.

Ces six formulations distinctes ont également été évaluées par un panel d'utilisateurs (Tableau 3). En particulier, les propriétés de résistance à la température et aux UV ont été appréciées dans le temps par ce panel d'utilisateurs. Le panel a pu également apprécier la qualité des autres propriétés, à savoir la tenue, la brillance, la couvrance et le temps de séchage ou le durcissement. Des notes ont été attribuées à chacune de ces formulations :
- 1 étant la note la plus faible et correspondant à un résultat médiocre,
- 5 étant la note la plus élevée et correspondant à un résultat excellent.
Les utilisateurs ont appliqué chaque composition sur leurs ongles afin d'en estimer leurs propriétés.

**Tableau 3**

| | **Base standard** | **Ex 1** | **Ex 2** | **Ex 3** | **Ex 4** | **Ex 5** |
|---|---|---|---|---|---|---|
| Propriétés sensorielles | 3 | 3,5 | 3,5 | 4 | 4 | 4 |
| Facilité d'étalement et résistance aux UV | 3 | 3,5 | 4 | 4,5 | 4,5 | 4,5 |
| Application | 3 | 3,5 | 4 | 4,5 | 4,5 | 3,5 |

Il a été observé pour les exemples 1-5 comprenant de la résine de Polyester-26 solubilisée dans de l'acétate de butyle obtiennent des notes supérieures en termes de facilité d'étalement et d'application et de décoloration beaucoup moins prononcée en comparaison avec la base standard. De surcroît, on peut constater que ces notes augmentent avec la quantité de résine de Polyester-26 solubilisée dans de l'acétate de butyle ajoutée. A partir d'une teneur en polyester-26 de 2,4 % en poids sec, le vernis à ongles commence à s'épaissir, ce qui va influencer la facilité d'étalement et l'application de celui-ci. Les propriétés de résistance aux UV restent néanmoins excellentes.

Il faut souligner également que l'ajout de la résine de Polyester-26 solubilisée dans de l'acétate de butyle n'a pas affecté les autres propriétés qui sont essentielles pour les utilisateurs à savoir, la brillance, la tenue, la couvrance ainsi que les temps de séchage et de durcissement.

Les résultats démontrent que l'ajout de résine de Polyester-26 solubilisée dans de l'acétate de butyle dans une composition anhydre pour vernis à ongles permet d'améliorer significativement sa résistance à la chaleur et à la lumière UV ainsi que ses propriétés d'étalement et d'application rendant sa pose facile et rapide.

## Revendications

1. Utilisation d'une résine de copolymère(s) d'acide adipique, d'acide fumarique, de néopentylglycol et de cyclohexanediméthanol comme agent(s) de protection contre les UV dans une composition anhydre de vernis à ongles.

2. Utilisation selon la revendication 1, dans laquelle la quantité de copolymère(s) d'acide adipique, d'acide fumarique, de néopentylglycol et de cyclohexanediméthanol est comprise entre 0,1 et 5% et de préférence entre 1 et 3% en poids sec par rapport au poids total de la composition anhydre.

3. Utilisation selon l'une des revendications 1 ou 2, dans laquelle la résine de copolymère(s) d'acide adipique, d'acide fumarique, de néopentylglycol et de cyclohexanediméthanol est la résine de Polyester-26 (désignation INCI).

4. Utilisation selon l'une quelconque des revendications 1 à 3, dans laquelle la résine de copolymère(s) d'acide adipique, d'acide fumarique, de néopentylglycol et de cyclohexanediméthanol, de préférence la résine de Polyester-26, est solubilisée dans de l'acétate de butyle.

5. Utilisation selon l'une quelconque des revendications 1 à 4, dans laquelle la composition anhydre comprend au moins un solvant organique cosmétiquement acceptable et au moins un agent filmogène.

6. Utilisation selon la revendication 5, dans laquelle l'agent filmogène est choisi parmi la cellulose, un dérivé de cellulose, une résine vinylique, une résine acrylique ou n'importe quelle combinaison de ceux-ci.

7. Utilisation selon l'une quelconque des revendications 5 ou 6, dans laquelle l'agent filmogène est la nitrocellulose.

8. Utilisation selon l'une quelconque des revendications 5 à 7, dans laquelle la quantité de l'agent filmogène est comprise entre 10 % et 20 % en poids sec par rapport au poids total de la composition anhydre.

9. Utilisation selon l'une quelconque des revendications 5 à 8, dans laquelle le solvant organique est choisi parmi le toluène, le xylène, l'acétate d'éthyle, l'acétate de butyle, l'acétate d'isobutyle, l'acétate de propyle, l'acétate de méthyle, une cétone, un ester, l'isopropanol, le butanol, le diacétone alcool, l'éthanol dénaturé avec la méthyléthylcétone ou le diéthyléther, les hydrocarbures linéaires ou cycliques, de préférence l'hexane, le cyclohexane, l'heptane ou n'importe quelle combinaison de ceux-ci.

10. Utilisation selon l'une quelconque des revendications 5 à 9, dans laquelle le solvant organique est l'acétate d'éthyle, l'acétate de butyle, de l'alcool ou une combinaison de ceux-ci.

11. Utilisation selon l'une quelconque des revendications 5 à 10, dans laquelle la quantité de solvant organique de la composition est comprise entre 60 % et 80 % par rapport au poids total de la composition anhydre.

12. Utilisation selon l'une quelconque des revendications 1 à 11, dans laquelle la composition anhydre comprend en outre un ou plusieurs additifs choisis parmi les résines, les plastifiants, les agents rhéologiques, les absorbeurs UV, les modificateurs de surface et les agents dits « traitants ».

13. Utilisation selon l'une quelconque des revendications 1 à 12, dans laquelle la composition anhydre comprend en outre un ou plusieurs agents de coloration choisis parmi les pigments, les nacres, les glitters et/ou les particules métalliques.
